(19) **Europäisches Patentamt**
**European Patent Office**
**Office européen des brevets**

(11) Veröffentlichungsnummer: **0 000 725**
**A1**

(12) **EUROPÄISCHE PATENTANMELDUNG**

(21) Anmeldenummer: **78100491.6**

(22) Anmeldetag: **24.07.78**

(51) Int. Cl.²: **A 61 N 1/04**

(30) Priorität: **16.08.77 DE 2736737**

(43) Veröffentlichungstag der Anmeldung:
**21.02.79 Patentblatt 79/4**

(84) Benannte Vertragsstaaten:
**BE CH DE FR GB LU NL SE**

(71) Anmelder: **Bisping, Hans-Jürgen, Dipl.-Ing.**
**Tittardshang 12**
**D-5100 Aachen-Laurensberg(DE)**

(72) Erfinder: **Bisping, Hans-Jürgen, Dipl.-Ing.**
**Tittardshang 12**
**D-5100 Aachen-Laurensberg(DE)**

(74) Vertreter: **Christiansen, Henning, Dipl.-Ing.**
**Baseler Strasse 172**
**D-1000 Berlin 45(DE)**

(54) Elektrode zur Implantation im Herzen.

(57) Elektrode zur Implantation im Herzen, insbesondere zur Stimulation des Herzmuskels, mit einer Elektrodenzuleitung, einer schraubenförmigen, vorstehenden Wendel am herznahen Ende der Elektrodenzuleitung zum Einschrauben der Elektrode ins Herzgewebe und einer die Front der Wendel während der Einführungsphase überragenden Schutzvorrichtung gegen ungewolltes Verhaken beim Einführen der Elektrode durch eine Vene, wobei innerhalb der schraubenförmigen Wendel (22) ein zylindrischer Körper (Schutzkern 23) relativ zu dieser in axialer Richtung derart bewegbar ist, dass er während der Einführungsphase der Elektrode mit der Front der schraubenförmigen Wendel (22) abschliesst oder diese überragt und zum Befestigen der schraubenförmigen Wendel (22) im Herzgewebe aus deren in das Gewebe einzuschraubenden Bereich mittels eines von ausserhalb des Körpers des Patienten betätigbaren Elements (28) entfernbar ist.

FIG. 6

1

Beschreibung

Die Erfindung betrifft eine Elektrode zur Implantation im Herzen, insbesondere zur Stimulation des Herzmuskels, mit einer Elektrodenzuleitung, einer schraubenförmigen, vorstehenden Wendel am herznahen Ende der Elektrodenzuleitung zum Einschrauben der Elektrode ins Herzgewebe und einer die Front der Wendel während der Einführungsphase überragenden Schutzvorrichtung gegen ungewolltes Verhaken.

Derartige Elektroden dienen zur Übertragung von Stimulationsimpulsen und physiologischen Signalen zwischen dem Herzmuskel und einem ebenfalls implantierten künstlichen Herzschrittmacher.

Dabei ist es aus der DE-OS 26 13 044 bekannt, die schraubenförmige Wendel durch eine diese seitlich umfassende elastische Manschette zu schützen. Wenn das herznahe Elektrodenende bei der Implantation an einer geeigneten Stelle innerhalb des Herzens plaziert ist, wird durch Ausübung einer Kraft in Einführungsrichtung über die Elektrodenzuleitung und ein gleichzeitiges Drehen derselben die Wendel in das Herzgewebe eingeschraubt, während der vordere Rand der Manschette im Bereich des die Einstichstelle umgebenden Gewebes aufliegt und sich mit dem Einschrauben der Wendel zunehmend zusammenstaucht, wodurch der einzuschraubende Teil der Wendel freigegeben wird. Hierzu muß
die Elektrodenzuleitung jedoch verhältnismäßig steif ausgebildet sei, so daß nicht nur der zum Einschrauben der
Wendel erforderliche, sondern zusätzlich auch der zum
Stauchen der Manschette notwendige Druck über die Elektrodenzuleitung übertragen werden kann. Aus Gründen der
Bruchsicherheit sollten derartige Elektrodenzuleitungen
aber möglichst weich sein. Weiterhin ist nachteilig, daß
die Manschette nach erfolgter Fixierung der Elektrode im
Herzgewebe ständig eine Kraft ausübt, die so gerichtet
ist, daß der eingeschraubte Teil des Elektrodenendes herausgezogen wird. Außerdem wird die Bildung von fibrinösem
Gewebe gefördert, so daß insgesamt die Tendenz besteht,
die Elektrode vorzeitig ineffektiv zu machen. Die Manschette muß dabei eine gewisse Mindeststeifigkeit aufweisen, da sie, wäre sie weicher ausgebildet, zwar im befestigten Zustand eine geringere Kraft ausüben würde, in
diesem Fall aber die Gefahr bestünde, daß die Manschette
die schraubenförmige Wendel schon während des Einführungsvorgangs freigibt, so daß sich diese in der Vene verhakt
oder daß sich die Manschette beim Einschrauben der Wendel
derart mit deren Gewindegängen oder Spitze verwickelt bzw.
verhakt, daß dadurch ein weiteres Einschrauben infolge
Blockierung nicht mehr möglich ist.

Ein weiterer Nachteil der genannten Elektrode besteht darin, daß sie bis zum Zeitpunkt der Fixierung von der Manschette umgeben ist, so daß vor dem Einschrauben ein elektrischer Kontakt mit dem Herzgewebe für Reizschwellenmessungen bzw. die Registrierung des intrakardialen Elektrokardiogramms nicht möglich ist.

Die Erfindung liegt die Aufgabe zugrunde, unter Vermeidung dieser Nachteile eine Elektrode der angegebenen Gattung so zu verbessern, daß sowohl ein gefahrloses Einführen als auch ein sicheres Befestigen im Herzgewebe gewährleistet ist. Vor der endgültigen Fixierung der Elektrode sollen elektrophysiologische Messungen vorgenommen werden können.

Diese Aufgabe wird erfindungsgemäß dadurch gelöst, daß innerhalb der schraubenförmigen Wendel ein zylindrischer Körper relativ zu dieser in axialer Richtung derart bewegbar ist, daß er während der Einführungsphase der Elektrode mit der Front der schraubenförmigen Wendel abschließt oder diese überragt und zum Befestigen der schraubenförmigen Wendel im Herzgewebe aus deren in das Gewebe einzuschraubenden Bereich mittels eines von außerhalb des Körpers des Patienten betätigbaren Elements entfernbar ist.

Der Erfindung liegt die Erkenntnis zugrunde, daß ein im wesentlichen zylindrischer Körper innerhalb des herausragenden Teils der Wendel der Elektrode derart gelagert werden kann, daß er durch eine einfache Bewegung, wie eine Zug-, Schub- oder Drehbewegung aus dem Eingriffsbereich der Wendel entfernbar ist. Eine derartige Bewegung kann durch ein innerhalb oder außerhalb der Elektrode geführtes unkompliziertes Betätigungselement aufgebracht werden. Die Beseitigung des als Schutzkern ausgebildeten zylindrischen Körpers, der einen sich an der Wendel abstützenden Kolben bildet, erfolgt durch eine relativ zwischen Betätigungselement und Elektrode aufgebrachte Kraft ohne Krafteinwirkung zwischen Elektrode und der den Einschraubbereich umgebenden Gewebeoberfläche.

Es ist in vorteilhafter Weise möglich, ohne Befestigung der schraubenförmige Wendel im Gewebe bereits Reizschwellenmessungen vorzunehmen, da die Schraubenwendel nach außen freiliegt und einen elektrischen Kontakt zur Gewebeoberfläche ermöglicht.

Der Antrieb des Körpers erfolgt ausschließlich über das Betätigungsmittel, und zwar durch Zug, Druck oder Drehung desselben. Für die Elektrodenzuleitung kann ein für derartige Elektrodenzuleitungen bevorzugter weicher Werkstoff Verwendung finden, da nicht zusätzlich zum Druck beim Einschrauben der Elektrode in das Herzgewebe noch eine Kraft zum Beseitigen der Schutzvorrichtung aufgebracht werden muß.

Dabei ist der Schutzkern vorteilhafterweise an einem entlang der Elektrodenleitung geführten Betätigungsfaden oder -draht befestigt. Damit wird der zylindrische Körper entsprechend in axialer Richtung angetrieben, so daß er während der Einführungsphase den einzuschraubenden Teil der schraubenförmige Wendel wirkungslos macht und vor Einschrauben der Wendel im Herzgewebe zurückgezogen werden kann.

In weiterer vorteilhafter Ausgestaltung ist der zylindrische Körper mindestens teilweise aus einem für Röntgenstrahlung undurchlässigem Material wie Platin hergestellt, so daß es dem implantierenden Arzt über ein Röntgengerät möglich ist, die Lage des zylindrischen Körpers in Bezug auf die Wendel jederzeit zu kontrollieren und sich damit davon zu überzeugen, daß der Eingriffsbereich der Wendel zum Einschrauben freigegeben ist.

Eine Erhöhung des Schutzes der schraubenförmige Wendel wird erreicht, wenn der zylindrische Körper an seinem vorderen Ende einen umlaufenden elastischen, gegebenenfalls als eine Art Lippe ausgebildeten Rand aufweist, der den zylindrischen Körper, d.h. den Eingriffsbereich der schraubenförmigen Wendel in radialer Richtung überragt.

Gemäß einer modifizierten Ausführung kann der zylindrische Körper tulpenförmig in seinem Inneren hohl ausgebildet sein, wodurch sich besonders günstige Möglichkeiten für den Aufbau ergeben.

Da die Wirksamkeit des zylindrischen Körpers von seiner Führung innerhalb der schraubenförmige Wendel abhängt, ist der Körper innerhalb vorzugsweise der Wendel im wesentlichen spielfrei gelagert bzw. weist gewindeähnliche Gänge auf, die im Zusammenwirken mit der schraubenförmigen Steigung der Wendel einen Schraubvorgang ermöglichen. Als "Schraubenzieher" kann dabei ein entsprechend ausgebildeter Führungsdraht dienen, der zum Versteifen der Elektrode beim Einführen ohnehin erforderlich ist. Da der Körper sich in Längsrichtung zweckmäßigerweise über mehrere Windungen der Wendel erstreckt, ergibt sich eine besonders stabile Führung für die Relativbewegung zwischen der Wendel und dem Körper.

Der Einführungsvorgang wird erleichtert, wenn der zylindrische Körper in seinem beim Einführen der Elektrode vorangeführten Bereich konvex verrundet ist.

Weitere vorteilhafte Ausführungsformen der Erfindung sind in den Unteransprüchen angegeben und werden im Folgenden anhand der Zeichnungen näher beschrieben. Es zeigen (jeweils vergrößert und im Schnitt dargestellt):

Fig.1 das mit dem Herzgewebe in Eingriff kommende Ende einer ersten Ausführungsform der erfindungsgemäßen Elektrode, wobei sich der zylindrische Körper in der die Wendel schützende Stellung befindet,

Fig. 2 die Ausführungsform gemäß Fig. 1, wobei sich der zylindrische Körper in zurückgezogener, die Wendel zum Einschrauben freigegebenen Stellung befindet,

Fig. 3 eine weitere Ausführungsform der erfindungsgemäßen Elektrode in der Darstellung entsprechend Fig. 1,

Fig. 4 die Ausführungsform der Elektrode gemäß Fig. 3 in der Fig. 2 entsprechenden Darstellung,

Fig. 5 eine Variante des zylindrischen Körpers für die Ausführungsform entsprechend den Fign. 3 und 4,

Fig. 6 eine dritte Ausführungsform der Erfindung, in der Fig. 1 entsprechenden Darstellung und

Fig. 7 die in Fig. 6 wiedergegebene Ausführungsform in der Fig. 2 entsprechenden Darstellung.

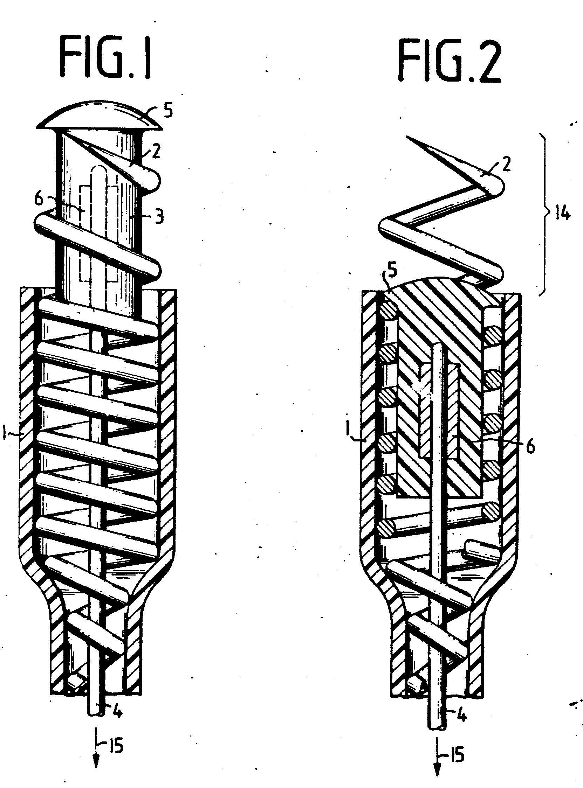

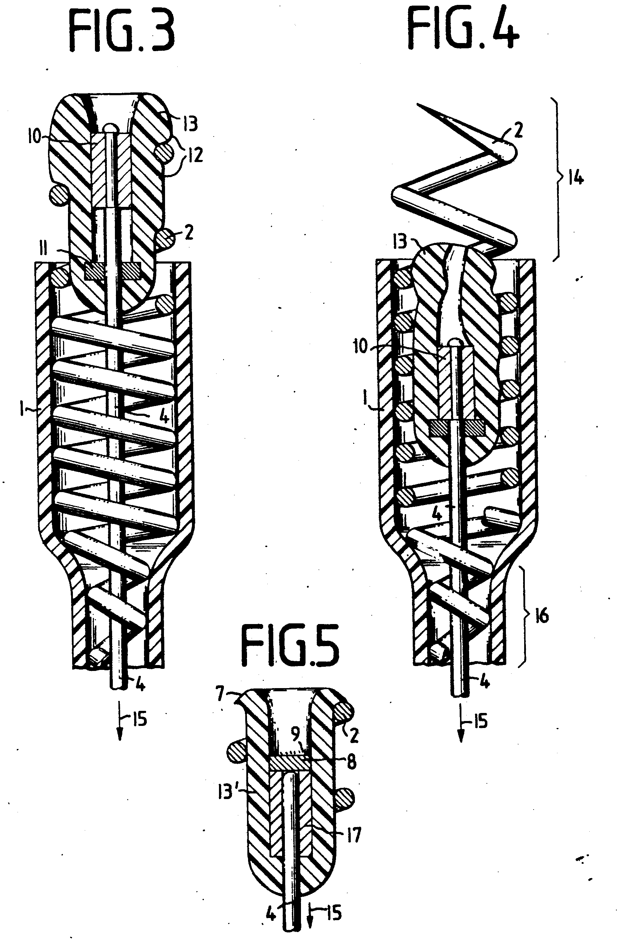

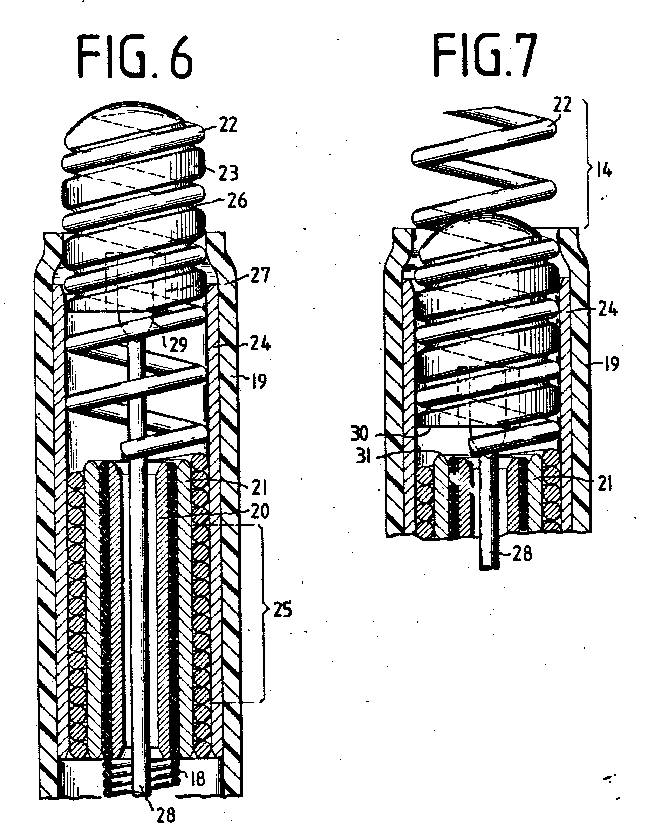

Bei dem in Fig. 1 dargestellten herzseitigen Ende einer ersten Ausführungsform der erfindungsgemäßen Elektrode besteht die Elektrodenzuleitung aus dem mit einem Mantel 1 aus flexiblem Material, wie beispielsweise Silikonkautschuk, überzogenen Teil einer schraubenförmigen Wendel 2. Dieser (in der Zeichnung gekürzt dargestellte) Teil weist die zur Verbindung des herzseitigen Endes der Elektrode mit dem implantierten Herzschrittmacher erforderliche Länge auf, wobei die Elektrodenzuleitung vorzugsweise in

/7

einer Vene verlegt wird. Der Mantel 1 läßt denjenigen korkenzieherartigen Bereich 14 (Fig. 2) der Wendel frei, der zum Befestigen des Endes in das Herzgewebe eingeschraubt wird. Der einzuschraubende Bereich 14 ist bei der hier dargestellten Ausführungsform, im Gegensatz zu der weiter unten beschriebenen, in den Fign. 5 und 6 dargestellten Ausführung einstückig mit dem die Zuleitung bildenden Teil der Wendel 2 ausgeführt.

Innerhalb der Wendel ist ein den zylindrischen Körper bildender Schutzkern 3 angeordnet, der nahezu spielfrei innerhalb der Wendel 2 in axialer Richtung beweglich gelagert ist. Der Schutzkern 3 ist zum Erleichtern des Einführens der Elektrode in seinem dabei vorangeführten Bereich konvex verrundet. Am Schutzkern 3 ist ein Betätigungsfaden 4 befestigt, der bis zum anderen Ende der Elektrodenzuleitung geführt ist, so daß der die Elektrode fixierende Arzt von dort aus eine Zugkraft in Richtung des Pfeils 15 auf den Schutzkern 3 ausüben kann, um ihn zurückzuziehen und zur Fixierung aus dem Bereich 14 der Wendel zu entfernen. Der in Einführungsrichtung der Elektrode vordere Bereich des Schutzkerns 3 hat einen größeren Durchmesser als die Wendel 2 an ihrem vorderen Ende und weist einen elastischen, umlaufenden Rand 5 auf, welcher den Eingriffsbereich der Wendel 2 in radialer Richtung so überragt, daß das als Spitze ausgebildete Ende derselben nicht mit dem Herzen in Eingriff kommen kann, bevor der Schutzkern 3 zurückgezogen ist. (Das Beseitigen des Schutzkerns 3 kann auch über ein starres Betätigungselement wie einen Draht durch Hinausschieben in entgegengesetzter Richtung erfolgen.) Der Schutzkern 3 selbst ist ebenfalls aus Silikonkautschuk hergestellt und enthält einen röntgendichten Kern (gestrichelt dargestellt), der als eine den Betätigungsfaden 4 sichernde Klemmhülse 6 ausgebildet und in den Schutzkern 3 einvulkanisiert ist.

Durch den röntgendichten Kern ist es möglich, jederzeit mittels eines Röntgengerätes während des Einführungsvorgangs die Lage des Schutzkerns 3 in Bezug auf die Wendel 2 im Herzen zu kontrollieren, so daß der Arzt sowohl die Lage des Elektrodenendes beurteilen kann als auch die Position des Kerns in Bezug auf die Wendel, d.h. ob die Wendel zum Einschrauben des Elektrodenendes den vorderen Teil der Wendel vollständig freigegeben hat. Um diese Freigabe zu bewirken, ist lediglich ein Zug an dem Sperrfaden 4 in Richtung des Pfeils 15 erforderlich, wobei der die Wendel 2 umhüllende Mantel 1 in einem außerhalb des Körpers des Patienten befindlichen Bereich der Elektrodenzuleitung festgehalten werden muß. Der Schutzkern 3 gelangt daraufhin in seine in Fig. 2 dargestellte Position. (Der Schutzkern ist hier im Gegensatz zu Fig. 1 geschnitten wiedergegeben.)

Die Länge des einen zylindrischen Körper bildenden Schutzkerns ist dabei so gewählt, daß eine sichere Führung desselben innerhalb der Wendel in Längsrichtung gewährleistet ist, was dadurch erreicht wird, daß er sich über mehrere Schraubengänge erstreckt. Durch die vorhandene Reibung zwischen dem Schutzkern 3 und der Wendel 2 werden unkontrollierte Relativbewegungen derselben unterbunden, die insbesondere während des Einführungsvorgangs zu verhindern sind, da sie eine vorzeitige Freigabe des Einschraubbereichs der Wendel zur Folge haben könnten.

Wie sich aus Fig. 2 ergibt, ist ein umlaufender elastischer Rand 5 derart nachgiebig ausgebildet, daß er sich beim Entfernen aus dem einzuschraubenen Bereich 14 der Wendel 2 durch Zug an dem Betätigungsfaden 4 derart verformt, daß er an den Windungen der Wendel vorbeigelangt. Dabei wird im wesentlichen nur jeweils der gerade die Schraubenlinie der Wendel schneidende Teilbereich des elastischen Rands 5 verformt, wie es in  Fig. 2 links ersichtlich ist.

In den Fign. 3, 4 und 5 ist eine zweite Ausführungsform der Erfindung in zwei Varianten dargestellt. Dabei ist der zylindrische Körper tulpenförmig ausgebildet, so daß sich durch den vorhandenen Innenhohlraum eine besonders gute Verformbarkeit der in die Wendel eingreifende Bereiche beim Entfernen des Körpers aus dem Eingriffsbereich der Wendel zum Einschrauben in das Herzgewebe ergibt. Bei der in den Fign. 3 und 4 dargestellten ersten Variante der zweiten Ausführungsform ist im inneren Hohlraum des Schutzkerns 13 eine mit dem Betätigungsfaden 4 verbundene Hülse 10 vorgesehen, die auf dem Betätigungsfaden 4 festgeklemmt ist. In der inneren Öffnung des tulpenförmig geformten Schutzkerns 13 ist die Hülse 10 in axialer verschiebbar angeordnet. In Betätigungsrichtung des Fadens 4 wird der Weg der Hülse 10 jedoch durch einen in den ebenfalls aus Silikonkautschuk bestehenden Schutzkern einvulkanisierten Anschlagring 11 begrenzt. Zum Einführen der Elektrode befinden sich die Hülse 10 und der Schutzkern 13 in der in Fig. 3 dargestellten Position. Die Außenoberfläche des Schutzkerns 13 weist eine schraubenförmig umlaufende Erhebung 12, die an die Wendel 2 in ihrem einzuschraubenden Bereich 14 angepaßt ist. Die Windungen der Wendel liegen dabei in der Rinne zwischen benachbarten Flanken der Erhebung 12, welche ein Zurückschieben des Schutzkerns 13 durch beim Einführen der Elektrode auftre-

/10

tende Kräfte mindestens insoweit verhindert, daß das scharfe Ende der Wendel 2 nicht über die vorangeführte Fläche des Schutzkerns hinausgelangt. Die Hülse 10 befindet sich im Bereich der in der Rinne gelagerten Windungen und verhindert ein elastisches Nachgeben der Wandung des hohlen Schutzkerns 13 nach innen, so daß die relative Lage von Schutzkern und Wendel aufrechterhalten bleibt, solange die Hülse 10 diese Position einnimmt.

Um den Eingriffsbereich 14 in das Herzgewebe einzuschrauben, zieht der behandelnde Arzt an dem Betätigungsfaden 4 in Richtung des Pfeils 15, woraufhin die auf dem Faden festgeklemmte Hülse 10 in dem Schutzkern 13 abwärts gleitet bis sie den Anschlagring 11 erreicht hat. Damit beginnt die Hülse 10 ihrerseits über den Anschlagring 11 den Schutzkern 13 in Richtung der Zugrichtung des Betätigungsfadens 4 anzutreiben. Da die Hülse sich nicht mehr in dem Bereich des Schutzkerns befindet, innerhalb dessen sie der elastischen Verformung des Wandbereichs, dadurch, daß der Eingriffsbereich 14 der Wendel in zwischen benachbarten Flanken der umlaufenden Erhebung 12 gelagert ist, einen Widerstand entgegensetzt, kann der Schutzkern hier seinen Querschnitt verringern und zwischen den Wendeln abwärts gleiten. Er nimmt dabei ein Profil ein, wie es in Fig. 4 dargestellt ist. Der Betätigungsfaden 4 kann dabei am anderen Ende der Elektrode soweit herausgezogen werden, bis der Schutzkern in einen anschließenden verengten Bereich 16 der Wendel gelangt, wo einer weiteren Bewegung in Richtung des Pfeils 15 ein Widerstand entgegengesetzt ist. Wenn die Hülse 10 aus einem für Röntgenstrahlen undurchlässigen Material gefertigt ist, kann der Arzt wiederum die Bewegung der Hülse in Bezug auf die Wendel mittels eines Röntgengerätes verfolgen.

0000725

In Fig. 5 ist eine Variante des Schutzkerns 13' dargestellt, der tulpenförmig ausgebildet ist. Der umlaufende Rand überragt die Wendel im Bereich der Frontebene des Eingriffsbereichs in radialer Richtung und ist elastisch deformierbar. Als Material wird ebenfalls Silikonkautschuk verwendet. Wenn auf den Betätigungsfaden 4 in Richtung des Pfeils 15 eine Kraft ausgeübt wird, deformiert sich der Rand in Richtung auf das Innere des Schutzkern 13' und gelangt dabei an der Wendel 2 vorbei. Innerhalb des Schutzkerns 13' ist wiederum ein röntgendichter Kern 17 angeordnet, der quetschend innerhalb des Schutzkerns 13' befestigt und mittels eines durch eine Klebstelle 9 befestigten Plättchens 8 gegen Herausfallen gesichert ist.

In den Fign. 6 und 7 ist eine weitere Ausführungsform der erfindungsgemäßen Elektrode dargestellt, wobei Fig. 6 den vorangeführten Teil der Elektrode im zum Einführen in die Vene bereiten Zustand zeigt. Die Elektrodenzuleitung besteht bei dieser Ausführungsform aus einer Zuleitungswendel 18, die von einem Silikonschlauch 19 ummantelt ist. In das Innere der Zuleitungswendel 18 ist eine innere Klemmhülse 20 eingefügt, während das Äußere der Zuleitungswendel 18 in diesem Bereich von einer mittleren Klemmhülse 21 umgeben ist. Um die mittlere Klemmhülse 21 ist wiederum die eigentliche Einschraubwendel 22 gewunden. Um diese Anordnung herum ist eine äußere Klemm- und Führungshülse 24 angeordnet, wobei die genannten Hülsen und Wendeln durch Quetschen im Bereich 25 mechanisch und elektrisch miteinander verbunden sind. Als Werkstoff für die Hülsen und Wendeln dient die Legierung "Elgiloy". Die Einschraubwendel 22 ragt mit ihrem Eingriffsbereich 14 aus dem Silikonschlauch 19 heraus.

/12

Während des Einführungsvorgangs befindet sich der Schutzkern 23 soweit außerhalb des Mantels 1, daß seine verrundete Frontfläche die Frontebene der Einschraubwendel 22 überragt. Der Schutzkern 23 weist eine ein Gewinde bildende umlaufende Rille 26 mit schraubenförmiger Steigung auf, die an die Steigung der Einschraubwendel 22 angepaßt ist. (Der Durchmesser der vordersten Wendel verkleinert sich spiralförmig zu ihrem herznahen Ende hin und ist damit der Verrundung des Schutzkerns angepaßt. Der Kern besteht aus im wesentlichen starren Material, wie beispielsweise Polyurethan. An seiner dem Inneren des Silikonschlauchs 19 zugewandten Ende ist eine gestrichelt dargestellte Ausnehmung 27 vorgesehen, mittels der über ein geeignetes Werkzeug nach Art einer Schraube/Schraubenzieher-Verbindung ein Drehmoment übertragen werden kann. Als Werkzeug zur Übertragung des Drehmoments wird ein Führungsdraht 28 benutzt, der ein abgeflachtes Ende 29 aufweist. Dieser Führungsdraht dient im übrigen zum Versteifen der Elektrodenzuleitung während des Einführungsvorgangs. Hat die Elektrode ihre für die Fixierung vorgesehene Position erreicht, so kann der Arzt mit dem noch seine Lage für den Einführungsvorgang einnehmenden Schutzkern vor der endgültigen Fixierung der Elektrode Schwellenmessungen vornehmen, da äußere Teile der Einschraubwendel 22 in ihrem Eingriffsbereich 14 bereits mit dem Gewebe in Kontakt kommen. Sind diese Messungen noch nicht zufriedenstellend, so kann ein weiteres Voranschieben oder Zurückziehen des Elektrodenendes beliebig erfolgen. Ist das Meßergebnis jedoch positiv, so wird mittels des in die Ausnehmung 27 eingreifenden abgeflachten Endes 29 des dabei um seine Achse gedrehten Führungsdrahtes 28 der Schutzkern 23 in das Innere des Silikonschlauches 19 zurückgeschraubt, so daß der Eingriffsbereich 14 der Einschraubwendel 22 ein

0000725

unbehindertes Einschrauben des Elektrodenendes in das Gewebe ermöglicht.

In Fig. 7 ist der Schutzkern 23 während des Zurückschraubens dargestellt. Er kann soweit in Richtung auf das Innere des Silikonschlauches 19 geschraubt werden, bis seine rückwärtige Fläche 30 mit der Kante 31 der mittleren Klemmhülse 21 in Kontakt kommt. Damit ergibt sich eine Bewegungsbegrenzung durch Anschlag, so daß für den Arzt erkennbar ist, daß der Schutzkern 23 in seine Endlage gelangt ist. Gleichzeitig liegt dabei die Fläche 21 an der Kante 30, welche die entsprechende Kante der inneren Klemmhülse 20 geringfügig überragt, dichtend an, so daß das Innere der Elektrodenzuleitung gegen eindringende Körperflüssigkeiten geschützt ist. Zum Einschrauben der Elektrode wird die Elektrodenzuleitung vom Arzt im Bereich des außerhalb des Körpers des Patienten befindlichen Endes gedreht. Anschließend wird der Führungsdraht 28 zurückgezogen.

/14

Bezugszeichenaufstellung

Mantel 1

schraubenförmige Wendel 2

Schutzkern 3

Betätigungsfaden 4

elastischer Rand 5

Klemmhülse 6

umlaufender Rand 7

Plättchen 8

Klebstelle 9

Hülse 10

Anschlagring 11

umlaufende Erhebung 12

Schutzkern 13, 13'

einzuschraubender Bereich 14

Pfeil 15

verengter Bereich 16

röntgendichter Kern 17

Zuleitungswendel 18

Silikonschlauch 19

innere Klemmhülse 20

mittlere Klemmhülse 21

Einschraubwendel 22

Schutzkern 23

äußere Klemm- und Führungshülse 24

Bereich 25

Rille 26

Ausnehmung 27

Führungsdraht 28

abgeflachtes Ende 29

rückwärtige Fläche 30

Kante 31

-.-.-.-.-

Patentansprüche

1. Elektrode zur Implantation im Herzen, insbesondere zu Stimulation des Herzmuskels, mit einer Elektrodenzuleitung, einer schraubenförmigen, vorstehenden Wendel am herznahen Ende der Elektrodenzuleitung zum Einschrauben der Elektrode ins Herzgewebe und einer die Front der Wendel während der Einführungsphase überragenden Schutzvorrichtung gegen ungewolltes Verhaken, d a d u r c h g e k e n n z e i c h n e t , daß innerhalb der schraubenförmigen Wendel (2) ein zylindrischer Körper (Schutzkern 3, 13, 13', 23) relativ zu dieser in axialer Richtung derart bewegbar ist, daß er während der Einführungsphase der Elektrode mit der Front der schraubenförmigen Wendel (2) abschließt oder diese überragt und zum Befestigen der schraubenförmigen Wendel (2) im Herzgewebe aus deren in das Gewebe einzuschraubenden Bereich (14) mittels eines von außerhalb des Körpers des Patienten betätigbaren Elements entfernbar ist.

2. Elektrode nach Anspruch 1, dadurch gekennzeichnet, daß Mittel zum Entfernen des zylindrischen Körpers (3, 13, 13', 23) aus dem in das Gewebe einzuschraubenden Bereich (14) in entgegengesetzter Richtung, bezogen auf die Einführungsrichtung der Elektrode, vorgesehen sind.

/2

3.  Elektrode nach einem der Ansprüche 1 oder 2, dadurch gekennzeichnet, daß der zylindrische Körper (3, 13, 13', 23) in seinem beim Einführen der Elektrode vorangeführten Bereich konvex verrundet ist.

4.  Elektrode nach einem der vorangehenden Ansprüche, dadurch gekennzeichnet, daß der zylindrische Körper (3, 13, 13', 23) im wesentlichen spielfrei innerhalb der schraubenförmigen Wendel (2) gelagert ist.

5.  Elektrode nach einem der vorangehenden Ansprüche, dadurch gekennzeichnet, daß der zylindrische Körper (3, 13, 13', 23) an seinem beim Einführen der Elektrode vorangeführten Ende einen umlaufenden, die schraubenförmige Wendel in ihrem in das Gewebe einzuschraubenden Bereich (14) in radialer Richtung mindestens teilweise überragenden, elastischen Rand (5, 7) aufweist.

6.  Elektrode nach Anspruch 5, dadurch gekennzeichnet, daß der zylindrische Körper (3, 13, 13', 23) im wesentlichen tulpenförmig ausgebildet ist, wobei sich die Öffnung an der beim Entfernen des Körpers rückwärtigen Seite desselben befindet.

7.  Elektrode nach einem der vorangehenden Ansprüche, dadurch gekennzeichnet, daß der zylindrische Körper (3, 13, 13', 23) an die Steigung der schraubenförmigen Wendel (2) nach Art von Gewindegängen angepaßte Erhebungs- und/oder Rillenbereiche (12) aufweist, die mit der schraubenförmigen Wendel (2) derart in Wirkverbindung treten können, daß ein Zurückschieben des zylindrischen Körpers entgegengesetzt zu Einführungsrichtung der Elektrode durch beim Einführen einwirkende Kräfte verhindert ist.

8. Elektrode nach einem der vorangehenden Ansprüchen, dadurch gekennzeichnet, daß der zylindrische Körper (3, 13, 13', 23) im Bereich der der Steigung der schraubenförmigen Wendel (2) angepaßten Erhebungsoder Rillenbereiche (12) derart nachgiebig ausgebildet ist, daß diese Bereiche durch Deformation des zylindrischen Körpers (3, 13, 13', 23) mit der schraubenförmigen Wendel (2) außer Eingriff gelangen können.

9. Elektrode nach Anspruch 8, dadurch gekennzeichnet, daß im wesentlichen innerhalb des zylindrischen Körpers (3, 13, 13', 23) ein in axialer Richtrung verschiebliches Element (Hülse 10) angeordnet ist, das, wenn es sich bei den Erhebungs- und Rillenbereichen (12) des zylindrischen Körpers befindet, ein Nachgeben desselben mindestens soweit verhindert, daß die Erhebungen bzw. Rillen mit der schraubenförmigen Wendel (2) in Wirkverbindung bleiben.

10. Elektrode nach einem der vorangehenden Ansprüche, dadurch gekennzeichnet, daß der zylindrische Körper (3, 13, 13', 23) an seiner beim Einführen der Elektrode rückwärtigen Seite (Fläche 30) eine Ausnehmung (27) aufweist, mittels der über ein weiteres Element durch Formschluß ein Drehmoment übertragbar ist.

11. Elektrode nach Anspruch 10, dadurch gekennzeichnet, daß als weiteres Element zur Übertragung eines Drehmoments ein im übrigen zur Versteifung der Elektrode beim Einführen dienender Führungsdraht (28) verwendbar ist.

12. Elektrode nach einem der vorangehenden Ansprüche, dadurch gekennzeichnet, daß der zylindrische Körper (3, 13, 13') mindestens mittelbar mit Hilfe eines entlang der Elektrodenzuleitung geführten Betätigungsfadens oder -drahts (4) aus dem in das Gewebe einzuschraubenden Bereich (14) der Wendel entfernbar ist.

13. Elektrode nach Anspruch 12, dadurch gekennzeichnet, daß der Betätigungsfaden oder -draht (4) an dem zylindrischen Körper (3, 13, 13') befestigt ist.

14. Elektrode nach den Ansprüchen 9 und 12, dadurch gekennzeichnet, daß der Betätigungsfaden oder -draht (4) an dem in axialer Richtung verschiebbaren Element (Hülse 10) befestigt ist, das seinerseits in dem zylindrischen Körper (13) um eine vorgegebene Weglänge in Betätigungsrichtung des Fadens oder Drahts verschiebbar ist.

15. Elektrode nach einem der vorangehenden Ansprüche, dadurch gekennzeichnet, daß der zylindrische Körper (3, 13, 13', 23) einen aus für Röntgenstrahlung undurchlässigem Material bestehenden Bereich (6, 10, 17) aufweist.

16. Elektrode nach einem der Ansprüche 2 bis 15, dadurch gekennzeichnet, daß der zylindrische Körper (23) nach dem Entfernen aus dem einzuschraubenden Bereich (14) der Wendel mit seiner in bezug auf die Einführungs richtung der Elektrode rückwärtigen Fläche (30) an einer das Innere der Zuleitung (Zuleitungswendel 18) umgebenden umlaufenden Kante (31) dichtend anliegt.

-.-.-.-.-

FIG.1

FIG.2

0000725

# FIG.3

# FIG.4

# FIG.5

0000725

# FIG. 6

# FIG. 7

**Europäisches Patentamt**

## EUROPÄISCHER RECHERCHENBERICHT

| | KLASSIFIKATION DER ANMELDUNG (Int.Cl.²) |
|---|---|

### EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | DE - A - 2 533 766 (BISPING) <br> * Seite 4, Abschnitt 3; Figur 1 * <br><br> -- <br><br> FR - A - 2 322 582 (OSYPKA) <br> * Seite 4, Zeilen 13-18; Seite 4, Zeile 36 bis Seite 5, Zeile 7 * <br><br> -- <br><br> FR - A - 2 302 107 (MEDTRONIC) <br> * Seite 7, Zeilen 25-32; Seite 9, Zeilen 29-32 * <br><br> ----- | 1,2,4, 7 <br><br><br><br> 1,10, 11 <br><br><br><br><br> 12,15 |

A 61 N 1/04

RECHERCHIERTE SACHGEBIETE (Int. Cl.³)

A 61 N 1/04

KATEGORIE DER GENANNTEN DOKUMENTE

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 9-11-1978 | SIMON |

EPA form 1503.1 06.78